# EUROPEAN PATENT SPECIFICATION

(11) **EP 3 884 916 B1**
(45) Date of publication and mention of the grant of the patent: **12.03.2025**
(21) Application number: 19902286.4
(22) Date of filing: 05.12.2019
(51) Int. Cl.: A61F 13/49, A61F 13/494, A61F 13/53, A61F 13/536, A61F 13/475, A61F 13/535

(54) **ABSORBENT ARTICLE**
ABSORBIERENDER ARTIKEL
ARTICLE ABSORBANT

(30) Priority: 29.12.2018 CN 201811634670
(43) Date of publication of application: 29.09.2021
(73) Proprietor: Unicharm Corporation, Shikokuchuo-shi, Ehime 799-0111 (JP)
(72) Inventor: TANGE, Satoru, Kanonji-shi, Kagawa 769-1602 (JP); GAO, Juyi, Shanghai, 201700 (CN); XU, Xingwang, Shanghai, 201700 (CN)
(74) Representative: Dolleymores
(86) International application number: PCT/JP2019/047690
(87) International publication number: WO 2020/137425

(56) References cited:
- EP-A1- 3 357 470
- WO-A1-2007/122938
- WO-A1-2011/162069
- WO-A1-2011/162069
- WO-A1-2012/043546
- JP-A- 2016 077 577
- JP-A- 2016 502 871
- JP-A- 2018 000 525
- JP-A- 2018 047 190
- JP-U- 3 218 599
- JP-U- 3 218 599

## Description

### FIELD

The present invention relates to an absorbent article.

### BACKGROUND

Absorbent articles in which an absorber containing a relatively large amount of superabsorbent polymers (SAP) as an absorbent material is used are known. The absorber containing a relatively large amount of superabsorbent polymers (so-called SAP sheet) has a thin thickness and is thus capable of enhancing wearability, fittability, or the like and can be said as a preferable absorber. As such an absorber, for example, Patent Literature 1 discloses a water absorbent sheet structure. The water absorbent sheet structure is used in an absorbent article, contains an absorbent resin as an absorbent material, and includes a wavy embossing that is provided in a central region in the widthwise direction, extends in a lengthwise direction and is interrupted at one or more points.

### [CITATION LIST]

### [PATENT LITERATURE]

### [Patent Literature 1]

Domestic Re-publication of PCT International Application No. 2012/043546

EP 3357470 A1 relates to an absorbent article comprising an absorbent element obtained by wrapping an absorbent body in a wrapping sheet.

WO 2011/162069 A1 discloses an absorbent article comprising a pair of leakage preventing walls.

JP 3218599 U provides a disposable diaper having a lattice compressed portion arranged in a lattice pattern on an absorbent core.

### SUMMARY

### [TECHNICAL PROBLEM]

The absorber containing a relatively large amount of superabsorbent polymers has a small thickness and low rigidity. Therefore, when such an absorber is applied to an absorbent article, there is a risk that the absorber is contracted in the lengthwise direction by a pair of (elastic members of) leak-proof walls that extend in the lengthwise direction along the absorber on both sides of the absorber in the widthwise direction. In such a case, the absorbent article is dragged by the contraction of the absorber, and the entire absorbent article is contracted in the lengthwise direction, thereby generating wrinkles in the absorbent article. Due to that, there is a risk of deterioration in the fitting properties or deterioration in the absorption performance.

The absorber (water absorbent sheet structure) of Patent Literature 1 has wavy embossings (compressed portions) that intermittently extend in the lengthwise direction, in the central region in the widthwise direction, in order to improve liquid permeability and the like. However, there is no consideration given to the possibility that the absorber may be contracted in the lengthwise direction by the pair of (elastic members of) leak-proof walls and that wrinkles may be generated in the entire absorbent article.

An object of the present invention is to provide an absorbent article containing superabsorbent polymers, which is capable of suppressing the generation of wrinkles and suppressing deterioration in fitting properties and deterioration in absorption performance.

### [SOLUTION TO PROBLEM]

The present invention provides the absorbent article of independent claim 1. The dependent claims specify preferred but optional features.

The absorbent article of the present invention is as follows. (1) An absorbent article having a lengthwise direction, a widthwise direction, and a thickness direction that are orthogonal to each other, and comprising: a stomach portion; a back portion; and an absorbent body that is positioned between the stomach portion and the back portion and includes an absorber, wherein the absorber contains superabsorbent polymers and includes a compressed portion that extends in the lengthwise direction, the absorbent body includes a pair of side sheets that are positioned on both sides in the widthwise direction and extend in the lengthwise direction on a surface on a skin side, the pair of side sheets each has fixed regions that are positioned at end portions of each of the side sheets on a front side and a back side in the lengthwise direction and fixed to a surface of the absorbent body, and a leak-proof wall that includes an elastic member extending along the lengthwise direction, positioned between the fixed regions on the front side and the back side of each of the side sheets in the lengthwise direction, and has an end edge on an outer side in the widthwise direction which is fixed to the surface of the absorbent body and an end edge on an inner side in the widthwise direction which is not fixed, and in the lengthwise direction, an end edge on the front side of the compressed portion is positioned on the front side with respect to an end edge on the front side of the leak-proof wall, and an end edge on the back side of the compressed portion is positioned on the back side with respect to an end edge on the back side of the leak-proof wall.

In the absorbent article, in the lengthwise direction, the end edge on the front side of the compressed portion is positioned on the front side with respect to the end edge on the front side of the leak-proof wall, and the end edge on the back side of the compressed portion is positioned on the back side with respect to the end edge on the back side of the leak-proof wall. As described above, since the compressed portion is formed so as to extend longer in the lengthwise direction than the leak-proof wall and to exist up to a position on the outer side in the lengthwise direction with respect to the leak-proof wall, the absorber can resist the contraction force of (the elastic member of) the leak-proof wall in the lengthwise direction due to the rigidity of the compressed portion. Thus, the absorber can be prevented from being contracted in the lengthwise direction, and the entire absorbent article can be prevented from being contracted in the lengthwise direction by being dragged by the contraction of the absorber. Therefore, the generation of wrinkles can be suppressed in the absorbent article and deterioration in fitting properties and deterioration in absorption performance can be suppressed.

A region that is formed by sandwiching the compressed portion between two straight lines parallel to each other in the lengthwise direction from both sides in the widthwise direction, includes the compressed portion, and extends in the lengthwise direction is defined as the compressed region. At this time, it can be considered that in order to increase the rigidity of the absorber to oppose the compressive force of the leak-proof wall in the lengthwise direction, the area of the compressed region may be increased. For example, in order to increase the area of the compressed region, the area (in a planar view) of the compressed portion may be increased. However, when the area of the compressed portion is excessively increased, there is a risk that the rigidity of the absorber becomes excessively high, that is, the absorber becomes excessively hard, which may deteriorate the fitting properties or increase the discomfort of the wearer. In an absorbent article which does not form part of the present invention, the compressed portion has a wavy shape that extends in the lengthwise direction. This makes it possible to increase the area of the compressed region while suppressing an increase in the area of the compressed portion. In other words, the area of the compressed region can be increased while suppressing an area ratio, which is the ratio of the area of the compressed portion to the area of the compressed region, at a low level. Therefore, the rigidity of the absorber can be increased to oppose the compressive force of the leak-proof wall in the lengthwise direction. Thus, the absorber can be prevented from being contracted in the lengthwise direction, and the entire absorbent article can be prevented from being contracted in the lengthwise direction by being dragged by the contraction of the absorber. Therefore, the generation of wrinkles can be suppressed in the absorbent article and deterioration in fitting properties and deterioration in absorption performance can be suppressed.

The compressed portion of the absorbent article of the present invention has an inclined grid shape formed of a plurality of rhombic shapes that are elongated in the lengthwise direction, the direction of the compressed portion being inclined with respect to the lengthwise direction. In the absorbent article, the compressed portion has an inclined grid shape formed of a plurality of rhombic shapes that are elongated in the lengthwise direction. Therefore, even when the compressive force of the leak-proof wall in the lengthwise direction is applied to the absorber, the direction of the compressed portion is inclined with respect to the direction of the compressive force, and thus, the contraction force can be laterally released and substantially reduced. In addition, although the direction of the compressed portion is inclined with respect to the direction of the compressive force, the direction is not perpendicular to the direction of the compressive force, and thus, the folding of the absorber can be suppressed with the compressed portion as a fold line. Therefore, the absorber can be further prevented from being contracted in the lengthwise direction.

In the widthwise direction, a part of the compressed portion is positioned in a region between the fixed regions on the front side and the back side in the lengthwise direction.

In the absorbent article, a part of the compressed portion is positioned in a region between the fixed region on the front side and the fixed region on the back side in the lengthwise direction, that is, in a region that overlaps the leak-proof wall in the thickness direction. Therefore, the absorber can more strongly resist the contraction force of the leak-proof wall in the lengthwise direction due to the rigidity of the compressed portion. Accordingly, the absorber can be further prevented from being contracted in the lengthwise direction.

The absorbent article of the present invention may be (2) the absorbent article according to the above (1), wherein in the widthwise direction, a part of the compressed portion is positioned on an outer side with respect to the non-fixed end edge on an inner side of the leak-proof wall.

In the absorbent article, a part of the compressed portion is positioned in a region on the outer side with respect to the non-fixed end edge on the inner side of the leak-proof wall, that is, in a region that further overlaps the leak-proof wall in the thickness direction. Therefore, the absorber can more strongly resist the contraction force of the leak-proof wall in the lengthwise direction due to the rigidity of the compressed portion. Therefore, the absorber can be further prevented from being contracted in the lengthwise direction.

The absorbent article of the present invention may be (3) the absorbent article according to the above (1) or (2), wherein the absorber has a rectangular shape in a planar view.

In a case where the absorber has an hourglass shape, that is, a shape having a portion narrowed in the widthwise direction at the central portion in the lengthwise direction, the contraction force in the lengthwise direction caused by the leak-proof wall is buffered, and thus, the absorber is less likely to be contracted in the lengthwise direction. However, in the absorbent article, since the absorber has a rectangular shape in a planar view, the contraction force of the leak-proof wall in the lengthwise direction makes it easier for the absorber to be contracted in the lengthwise direction. That is, there is a risk that wrinkles are generated in the absorbent article to deteriorate the fitting properties and deteriorate the absorption performance of the absorbent article. However, in the absorbent article, since the compressed portion is formed so as to extend longer in the lengthwise direction than at least the leak-proof wall, due to the rigidity of the compressed portion, the absorber can resist the contraction force of the leak-proof wall in the lengthwise direction, which makes it possible to prevent the absorber from being contracted in the lengthwise direction.

The absorbent article of the present invention may (4) the absorbent article according to any one of the above (1) to (3), further comprising: a crotch portion positioned between the stomach portion and the back portion, wherein the crotch portion includes leg gathers in portions that surround both leg portions of a wearer, and a contraction force of the leak-proof wall is lower than a contraction force of the leg gathers.

In the absorbent article, since the contraction force of the leak-proof wall is lower than the contraction force of the leg gathers, the absorber can further resist the contraction force of the leak-proof wall due to the rigidity of the compressed portion in the lengthwise direction, which makes it possible to further prevent the absorber from being contracted in the lengthwise direction.

The absorbent article of the present invention may (5) the absorbent article according to any one of the above (1) to (4), wherein the absorber includes: an absorbent core containing superabsorbent polymers; a first core wrap that covers a surface on a skin side of the absorbent core, and a second core wrap that covers a surface on a non-skin side of the absorbent core, each of both end portions of the first core wrap and each of both end portions of the second core wrap in the widthwise direction are joined in a joining portion extending along the lengthwise direction, the joining portion is positioned on one of the surface on the skin side or the surface on the non-skin side of the absorbent core, and the joining portion overlaps a part of the compressed portion in the thickness direction.

In the absorbent article, the joining portion overlaps the compressed portion in the thickness direction. Therefore, the rigidity of the compressed portion can be further increased. Therefore, the absorber can further resist the contraction force of the leak-proof wall in the lengthwise direction due to the rigidity of the compressed portion, which makes it possible to further prevent the absorber from being contracted in the lengthwise direction.

The absorbent article of the present invention may be (6) the absorbent article according to any one of the above (1) to (5), wherein the absorber has at least one channel extending along the lengthwise direction.

In the absorbent article, the absorber has at least one channel extending along the lengthwise direction. Thus, the rigidity of the absorber in the lengthwise direction can be further increased. Therefore, the absorber can further resist the contraction force of the leak-proof wall in the lengthwise direction, which makes it possible to further prevent the absorber from being contracted in the lengthwise direction.

The absorbent article of the present invention may be (7) the absorbent article according to any one of the above (1) to (6), wherein the absorbent body includes a top sheet positioned on a skin side of the absorber, and the top sheet includes: a plurality of protruding portions extending along the lengthwise direction and arranged side by side at intervals, and a plurality of recessed portions each positioned between adjacent protruding portions of the plurality of protruding portions.

In the absorbent article, the top sheet includes the plurality of protruding portions and the plurality of recessed portions that extend in the lengthwise direction. Thus, the rigidity of the absorbent body in the lengthwise direction can be increased. Therefore, by adding the rigidity of the top sheet, the absorber can resist the contraction force of the leak-proof wall in the lengthwise direction, which makes it possible to further prevent the absorber from being contracted in the lengthwise direction.

### [ADVANTAGEOUS EFFECT OF THE INVENTION]

According to the present invention, an absorbent article containing superabsorbent polymers, which is capable of suppressing the generation of wrinkles and suppressing deterioration in fitting properties or deterioration in absorption performance.

### BRIEF DESCRIPTION OF DRAWINGS

FIG. 1 is a plan view showing a configuration example of a pants-type diaper according to an embodiment.
FIG. 2 is a cross-sectional view taken along a line II-II in FIG. 1.
FIG. 3 is a plan view and a cross-sectional view showing a configuration example of an absorber according to the embodiment.
FIG. 4 is a plan view showing a configuration example of the absorbent body according to the embodiment.
FIG. 5 is an enlarged plan view of a part of a back portion of the pants-type diaper according to the embodiment.
FIG. 6 is an enlarged plan view of a part of a stomach portion of the pants-type diaper according to the embodiment.

### DESCRIPTION OF EMBODIMENTS

Hereinafter, absorbent articles according to embodiments will be described using an underpants-type disposable diaper (hereinafter, also simply referred to as a pants-type diaper). However, the absorbent article is not limited to this example, and other types of absorbent articles may be used without departing from the scope of the subject matter of the present invention. Examples of such absorbent articles include a three-piece type disposable diaper and a tape-type disposable diaper.

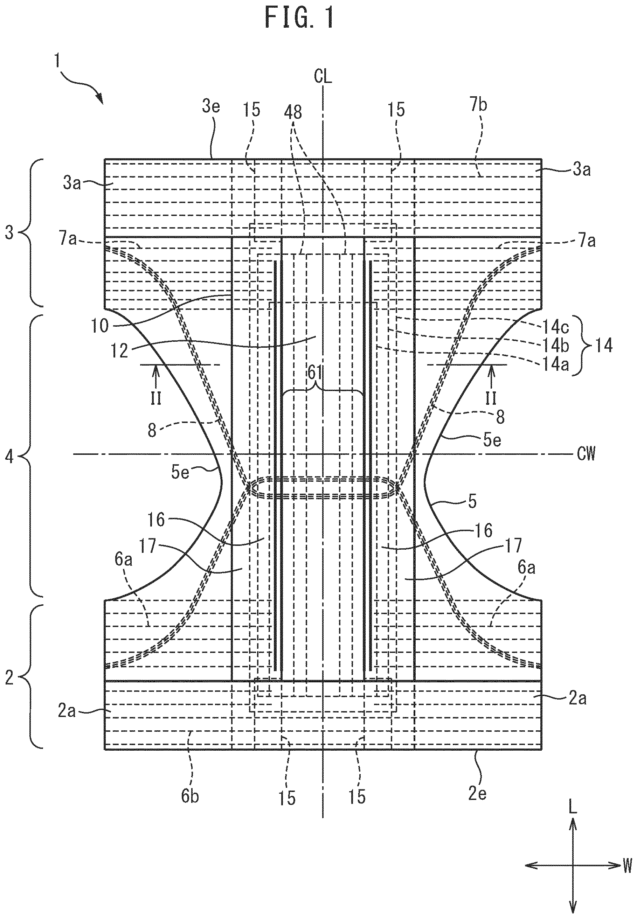

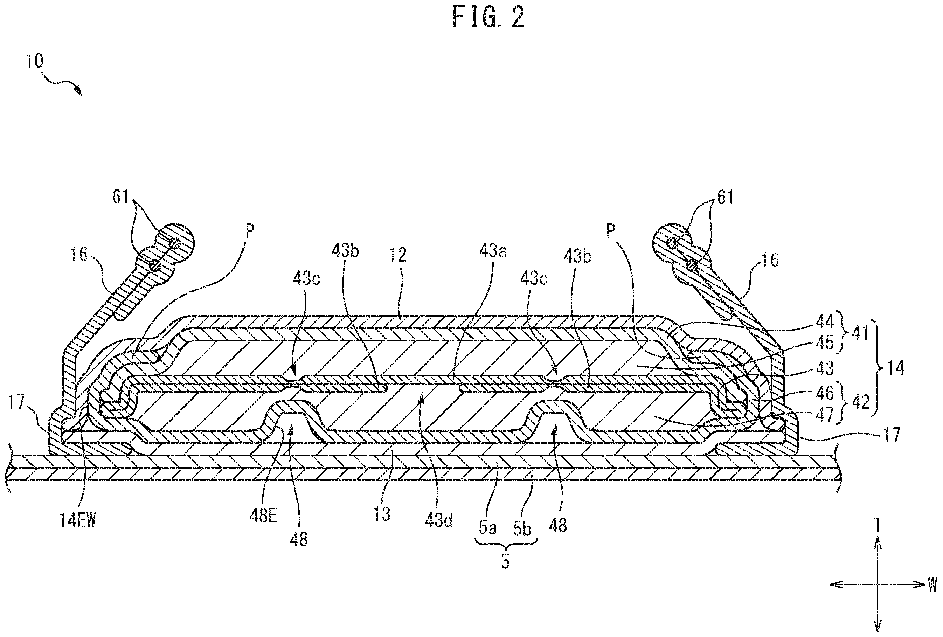

FIGS.1 and 2 are view showing configuration examples of a pants-type diaper 1 according to an embodiment. FIG. 1 is a plan view showing the pants-type diaper 1 in an unfolded state, and FIG. 2 is a cross-sectional view taken along a line II-II in FIG. 1. In the state shown in FIG. 1, the pants-type diaper 1 has a lengthwise direction L, a widthwise direction W, and a thickness direction T that are orthogonal to each other, and has a lengthwise direction center line CL passing through the center of the widthwise direction W and extending in the lengthwise direction L and a widthwise direction center line CW passing through the center of the lengthwise direction L and extending in the widthwise direction W. An orientation and a side toward the lengthwise direction centerline CL are defined as inward and the inner side in the widthwise direction W, respectively, and an orientation and a side away from the lengthwise direction centerline CL are defined as outward and the outer side in the widthwise direction W, respectively. An orientation and a side toward the widthwise direction centerline CW are defined as inward and the inner side in the lengthwise direction L, respectively, and an orientation and a side away from the widthwise direction centerline CW are defined as outward and the outer side in the lengthwise direction L, respectively. Further, in the lengthwise direction L, a side facing an end edge of the pants-type diaper 1 corresponding to the stomach part of the wearer (an end edge on the stomach side) will also be referred to as a front side in the lengthwise direction L, and a side facing an end edge of the pants-type diaper 1 corresponding to the back of the wearer (an end edge on the back side) will also be referred to as a back side in the lengthwise direction L. The term "planar view" refers to a state in which the pants-type diaper 1 in a state of being unfolded in a plane including the lengthwise direction L and the widthwise direction W is viewed from above in the thickness direction T, and the term "planar shape" refers to a shape grasped in a planar view. "Plane direction" is any direction parallel to the plane including the widthwise direction W and the lengthwise direction L. The terms "skin side" and "non-skin side" mean sides that, when the pants-type diaper 1 is worn by a wearer, are relatively close to and far from the skin surface of the wearer in the thickness direction T, respectively. These definitions are commonly used not only for the pants-type diaper 1, but also for the absorber of the pants-type diaper 1 and each material arranged thereon.

It should be noted that a member, a structure, a shape, or the like being along the lengthwise direction L indicates not only a case where the member or the like is parallel to the lengthwise direction L but also a case where a component Dx of the member or the like in the lengthwise direction L is larger than a component Dy of the member or the like in the widthwise direction W (Dx > Dy). Similarly, the member or the like being along the widthwise direction W indicates not only a case where the member or the like is parallel to the widthwise direction W but also a case where the component Dy of the member or the like in the widthwise direction W is larger than the component Dx of the member or the like in the lengthwise direction L (Dy > Dx). Regarding the member or the like having a curved line shape or a curved surface shape, the member or the like is evaluated as described above using the tangent line at each point on the curved line or the like.

As shown in FIG. 1, the pants-type diaper 1 includes, in the lengthwise direction L, a stomach portion (stomach waist belt) 2, a back portion (back waist belt) 3, and an absorbent body 10 positioned between the stomach portion 2 and the back portion 3. In the first embodiment, the pants-type diaper 1 further includes a crotch portion 4 located between the stomach portion 2 and the back portion 3. The stomach portion 2 is a portion that comes into contact with the wearer's abdomen. The back portion 3 is a portion that comes into contact with the wearer's buttock portion or back portion. The absorbent body 10 is a portion that comes into contact with the wearer's crotch, one end portion in the lengthwise direction L is laminated on the stomach portion 2, and the other end portion is laminated on the back portion 3, respectively. The crotch portion 4 is a portion that supports the absorbent body 10 from the non-skin side. Both end portions 2a and 2a of the stomach portion 2 in the widthwise direction W and both end portions 3a and 3a of the back portion 3 in the widthwise direction W respectively overlap in the thickness direction T and are joined along the lengthwise direction L, thereby forming the pants-type diaper 1. In this case, in the pants-type diaper 1, a waist opening through which the waist of the wearer passes is defined by an end portion 2e of the stomach portion 2 on the outer side in the lengthwise direction L and an end portion 3e of the back portion 3 on the outer side in the lengthwise direction L. Further, in the pants-type diaper 1, both side portions 5e and 5e on both sides in the widthwise direction W in the crotch portion 4 define a pair of leg openings through which legs of the wearer pass. It should be noted that the stomach portion (stomach waist belt) 2 and the back portion (back waist belt) 3 can be said to be portions that are demarcated by ranges in the lengthwise direction L in which both end portions 2a and 2a and both end portions 3a and 3a are joined to each other.

In the present embodiment, in the state illustrated in FIG. 1, the stomach portion 2 and the back portion 3 each has a rectangular shape extending substantially in the widthwise direction W and are spaced apart from each other in the lengthwise direction L. The crotch portion 4 is located between the stomach portion 2 and the back portion 3, and both side edges in the widthwise direction W are recessed inward in the widthwise direction W. The stomach portion 2, the crotch portion 4, and the back portion 3 are integrally formed with each other. In another embodiment, the stomach portion 2, the crotch portion 4, and the back portion 3 are separately formed from each other. Furthermore, in another embodiment (not shown), the pants-type diaper 1 includes the stomach portion 2 and the back portion 3, and does not include the crotch portion 4.

In the present embodiment, the stomach portion 2, the back portion 3, and the crotch portion 4 include a liquid-impermeable cover sheet (sheet member) 5. The cover sheet 5 includes a cover sheet 5a that is located on the skin side and a cover sheet 5b that is located on the non-skin side. The cover sheet 5a and the cover sheet 5b are laminated together in the thickness direction T and joined to each other with an adhesive or the like. Two end portions of the cover sheet 5b in the lengthwise direction L are folded back toward the skin so as to cover both end portions of the cover sheet 5a in the lengthwise direction L. In that case, the cover sheet 5b at the folded-back locations in the stomach portion 2 and the back portion 3 configures the end portion 2e of the stomach portion 2 and the end portion 3e of the back portion 3, respectively.

Examples of the cover sheet 5 include any liquid-impermeable sheets such as a liquid-impermeable non-woven fabric, a synthetic resin film, a composite sheet thereof, an SB non-woven fabric, and an SMS non-woven fabric. Examples of the material of the cover sheet 5 include polyolefin-based materials such as polypropylene or polyethylene. The basis weight of the cover sheet 5 is, for example, 5 to 100 g/m² and preferably 10 to 50 g/m². The dimension of the cover sheet 5 in the thickness direction T (thickness) is, for example, 0.1 to 5 mm and preferably 0.1 to 2 mm. In another embodiment, the number of the cover sheets 5 is one or three or more (not shown). In still another embodiment, the cover sheet 5b is not folded back (not shown).

In the present embodiment, the stomach portion 2 and the back portion 3 include a plurality of elastic members 6a and 6b and a plurality of elastic members 7a and 7b for waist gather, respectively, between the cover sheet 5a and the cover sheet 5b. The plurality of elastic members 6a and 6b are arranged in the outer side and inner side of the stomach portion 2 in the lengthwise direction L, respectively. The plurality of elastic members 6a are arranged at intervals in the lengthwise direction L along the widthwise direction W in individual predetermined regions on both sides across the longitudinal centerline CL. The predetermined region is a region from the end portion 2a to the inner portion of the end edge of the absorbent body 10 in the widthwise direction W that faces the end portion 2a. The plurality of elastic members 6b extend from one end portion 2a to the other end portion 2a along the widthwise direction W and are arranged at intervals in the lengthwise direction L. Similarly, the plurality of elastic members 7a and 7b are arranged in the outer side and inner side of the back portion 3 in the lengthwise direction L, respectively. The plurality of elastic members 7a are arranged at intervals in the lengthwise direction L along the widthwise direction W in individual predetermined regions on both sides across the longitudinal centerline CL. The predetermined region is a region from the end portion 3a to the inner portion of the end edge of the absorbent body 10 in the widthwise direction W that faces the end portion 3a. The plurality of elastic members 7b extend from one end portion 3a to the other end portion 3a along the widthwise direction W and are arranged at intervals in the lengthwise direction L. The plurality of elastic members 6a, 6b, 7a, and 7b stretch and contract the waist opening and are exemplified by rubber threads. It can be also said that the stomach portion (stomach waist belt) 2 and the back portion (back waist belt) 3 are portions that are demarcated by ranges in the lengthwise direction L in which the plurality of elastic members 6a, 6b, 7a, and 7b are arranged.

In the present embodiment, the pants-type diaper 1 includes a plurality of elastic members 8 for leg gathering (leg gathering) from the crotch portion 4 to the back portion 3 and the stomach portion 2. The plurality of elastic members 8 are provided to extend along the lengthwise direction L mainly at both end portions of the crotch portion 4 in the widthwise direction W. The plurality of elastic members 8 stretch and contract each of the pair of leg openings and are exemplified by rubber threads. The contraction force of a leak-proof wall 16, that is, the contraction force of the plurality of elastic members 61, is lower than the contraction force of the leg gather, that is, the contraction force of the plurality of elastic members 8. Therefore, even in a case where the rigidity of the absorber 14 is relatively low, the absorber 14 can oppose the contraction force of the leak-proof wall 16 (plurality of elastic members 61).

In the present embodiment, the absorbent body 10 has a substantially rectangular shape and includes a liquid-permeable top sheet 12, a liquid-impermeable back sheet 13, and an absorber 14 that is located between the top sheet 12 and the back sheet 13 and absorbs and retains liquid. Examples of the top sheet 12 include a liquid-permeable non-woven fabric or woven fabric, a synthetic resin film having a liquid-permeable hole formed therein, a composite sheet thereof, and the like. The top sheet 12 may be a sheet that includes a plurality of protruding portions extending in the lengthwise direction L and arranged side by side at intervals and a plurality of recessed portions each positioned between adjacent protruding portions among the plurality of protruding portions, and has the protruding portions and recessed portions extending along the lengthwise direction L on the surface. In such a case, the rigidity of the absorbent body 10 in the lengthwise direction L can be increased. Examples of the back sheet 13 include a liquid-impermeable non-woven fabric or synthetic resin film, a composite sheet thereof, an SMS non-woven fabric, and the like. In the present embodiment, the absorber 14 includes an absorbent core that absorbs and retains liquid and a core wrap that encloses the absorbent core. A detail of the absorber 14 will be described below. The absorber 14 and the top sheet 12, and, the absorber 14 and the back sheet 13 are joined together with an adhesive, respectively, and the top sheet 12 and the back sheet 13 are joined together with an adhesive at the peripheral edge portions. The adhesive includes materials well-known in pants-type diapers, for example, hot melt adhesives. The shape of the absorbent body 10 is not limited to the above-described example as long as the shape is long in the lengthwise direction L, and examples thereof include a rectangular shape with rounded corners and a rectangular shape or hourglass shape in which a short side is a curved line that is convex outward. In addition, in another embodiment, the back sheet 13 is not provided, and the surface on the non-skin side of the absorber 14 and the surface at the peripheral edge portion on the non-skin side of the top sheet 12 are joined to the cover sheet 5.

In the present embodiment, the absorbent body 10 includes a pair of side sheets 17 and 17 that are located on both sides in the widthwise direction W and extend in the lengthwise direction L on the skin-side surface. Each side sheet 17 has a leak-proof wall 16 and fixation regions 15 and 15. The fixation regions 15 and 15 are located at the end portions on the front and back side of the side sheet 17 in the lengthwise direction L and are fixed to the surface on the skin-side of the absorbent body 10. The leak-proof wall 16 is located between the fixation regions 15 and 15 in the lengthwise direction L of the side sheet 17, is adjacent to the fixation regions 15 and 15, and is fixed to the surface on the skin-side of the absorbent body 10 at an outer end edge in the widthwise direction W, but remains unfixed at an inner end edge in the widthwise direction W. In this case, the leak-proof wall 16 and the fixation regions 15 and 15 are formed at, for example, the inner portion of the side sheet 17 in the widthwise direction W, and the outer portion of the side sheet 17 in the widthwise direction W is fixed to the absorbent body 10. As described above, the absorbent body 10 includes a pair of the leak-proof walls 16 and 16. The pair of leak-proof walls 16 and 16 are arranged at both end portions of the absorbent body 10 in the widthwise direction W on the skin side so as to face each other and continuously extend along the lengthwise direction L. Each leak-proof wall 16 includes two elastic members 61 extending along the lengthwise direction L at the inner end portion in the widthwise direction W. The elastic member 61 is exemplified by a rubber thread. In another embodiment, the pair of leak-proof walls 16 and 16 are each folded back further outward in the widthwise direction W at the inner end portion in the widthwise direction W (described below). In another embodiment, the number of the elastic members 61 is one or three or more. The pair of leak-proof walls 16 and 16 are each formed of a hydrophobic sheet, for example, a hydrophobic non-woven fabric. In another embodiment, the leak-proof wall 16 is formed of a hydrophilic sheet, for example, a hydrophilic non-woven fabric.

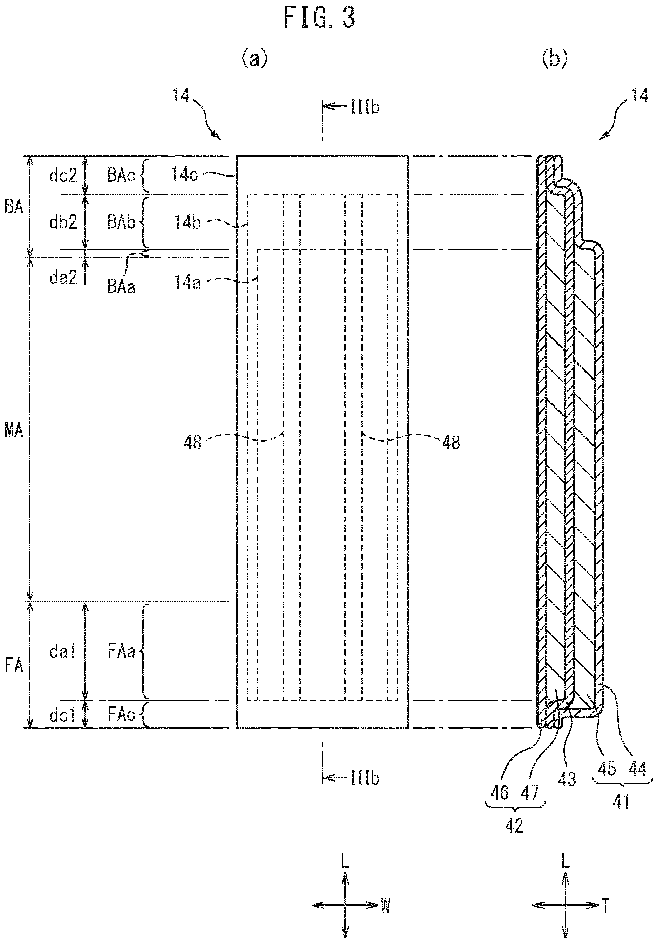

Next, the absorber 14 will be described with reference to FIG. 1 to FIG. 3. Here, FIG. 3 is a diagram illustrating a configuration example of the absorber 14 according to the embodiment, FIG. 3(a) is a plan view, and FIG. 3(b) is a cross-sectional view taken along a line IIIb-IIIb in FIG. 3(a). The absorber 14 is a layer having liquid absorption performance and liquid retention performance and, in the present embodiment, includes a first absorbing layer 41 that is located on the skin side, a second absorbing layer 42 that is located on the non-skin side, and an intermediate layer 43 that is located between the first absorbing layer 41 and the second absorbing layer 42. The absorber 14 has a two-layer structure in which the first absorbing layer 41, the intermediate layer 43, and the second absorbing layer 42 are laminated in this order in the thickness direction T. It should be noted that, in another embodiment, the absorber 14 has a single-layer structure in which the intermediate layer 43 is not present and the first absorbing layer 41 and the second absorbing layer 42 are integrated together in the thickness direction. In still another embodiment, the absorber 14 has a multiple (three or more)-layer structure in which another intermediate layer and another absorbing layer are further laminated between the first absorbing layer 41 and the second absorbing layer 42. In another embodiment, the first absorbing layer 41 is located on the non-skin side, and the second absorbing layer 42 is located on the skin side.

In the present embodiment, the absorber 14 has a substantially rectangular planar shape extending in the lengthwise direction L. It can be seen that the absorber 14 has the first water absorbing material 45 and the second water absorbing material 47 as the absorbing core, and has the first base material 44 and the second base material 46 as the core wrap. However, the shape is not particularly limited, and examples thereof include a rectangular shape having a short side that protrudes in an arc shape, a rectangular shape with rounded corners, an ellipse, and an hourglass. The thickness of the absorber 14 is, for example, 0.5 to 20 mm and preferably 1 to 10 mm. The basis weight of the absorber 14 can be appropriately adjusted depending on absorption performance required for the pants-type diaper 1 and is, for example, 60 to 1600 g/m².

In the present embodiment, the first absorbing layer 41 includes the first base material 44 formed of a liquid-permeable sheet and the first water absorbing material 45 that is arranged on the intermediate layer 43 side with respect to the first base material 44 and has a water absorbing material containing water absorbing polymers. It can be seen that the first absorbing layer 41 has the first water absorbing material 45 as the absorbing core and the first base material 45 (and the intermediate layer 43) as the core wrap. It should be noted that, in another embodiment, as the core wrap, for example, a tissue core wrap sheet enclosing the first water absorbing material 45 is provided separately from the first base material 44 (and the intermediate layer 43) inside thereof. The first water absorbing material 45 is fixed to at least one of the first base material 44 and the intermediate layer 43 with an adhesive used to coat at least one of the intermediate layer 43-side surface of the first base material 44 and the first base material 44-side surface of the intermediate layer 43. A water absorbing material of the first water absorbing material 45 is arranged in the first absorbing layer 41 in a substantially uniform basis weight. However, the basis weight may gradually decrease toward the end edges at the end portions in the lengthwise direction L and/or in the widthwise direction W. In this case, the end portion is, for example, a region as large as 10% or less of the maximum dimension in the lengthwise direction L and in the widthwise direction W at either end in the lengthwise direction L and in the widthwise direction W. In another embodiment, the water absorbing material of the first water absorbing material 45 is arranged in the first absorbing layer 41 in a predetermined distribution having a portion with a large basis weight and a portion with a small basis weight. The first water absorbing material 45 has a substantially rectangular planar shape. The first base material 44 has a substantially rectangular planar shape and covers the first water absorbing material 45 from the skin side in a planar view, and the peripheral edge portion of the first base material 44 slightly extend outward from the periphery of the first water absorbing material 45. As the outer dimension (outer edge) of the first absorbing layer 41 in the plane direction, for example, the outer dimension (outer edge) of the first water absorbing material 45 in the plane direction is used. The outer dimension (outer edge) is measured at, for example, the halfway location of the first water absorbing material 45 in the thickness direction T.

In the present embodiment, the second absorbing layer 42 includes the second base material 46 formed of a water-retaining and liquid-diffusing sheet and the second water absorbing material 47 that is arranged on the intermediate layer 43 side with respect to the second base material 46 and has a water absorbing material containing water absorbing polymers. It can be seen that the second absorbing layer 42 has the second water absorbing material 47 as the absorbing core and the second base material 46 (and the intermediate layer 43) as the core wrap. It should be noted that, in another embodiment, as the core wrap, for example, a tissue core wrap sheet enclosing the second water absorbing material 47 is provided separately from the second base material 46 (and the intermediate layer 43) inside thereof. The second water absorbing material 47 is fixed to at least one of the second base material 46 and the intermediate layer 43 with an adhesive used to coat at least one of the intermediate layer 43-side surface of the second base material 46 and the second base material 46-side surface of the intermediate layer 43. A water absorbing material of the second water absorbing material 47 is arranged in the second absorbing layer 42 except a pair of channels 48 and 48 (described below) in a substantially uniform basis weight. However, the basis weight may gradually decrease toward the end edges at the end portions in the lengthwise direction L and/or in the widthwise direction W. In this case, the end portion is as described above. In another embodiment, the water absorbing material of the second water absorbing material 47 is arranged in the second absorbing layer 42 in a predetermined distribution having a portion with a large basis weight and a portion with a small basis weight. The second water absorbing material 47 has a substantially rectangular planar shape that is slightly larger than the first water absorbing material 45 in the widthwise direction W and in the lengthwise direction L. The second base material 46 has a substantially rectangular planar shape and covers the second water absorbing material 47 from the non-skin side in a planar view, and the peripheral edge portion of the second base material 46 slightly extend outward from the periphery of the second water absorbing material 47. At that time, both end portions of the second base material 46 in the widthwise direction W cover both side surfaces of the second water absorbing material 47 and cover the skin-side surface of the first base material 44 at both end portions in the widthwise direction W. That is, on the surface on the skin-side of the first absorbing layer 41 at the end portions in the widthwise direction W, the end portions of the second base material 46 in the widthwise direction W and the end portions of the first base material 44 in the widthwise direction W are overlapped and joined together. Therefore, when the end portion of the second base material 46 overlapping with the first base material 44 in the widthwise direction W is an overlapping portion P, the second base material 46 has a pair of overlapping portions P and P that extend along the lengthwise direction L at both end portions in the widthwise direction W. As the outer dimension (outer edge) of the second absorbing layer 42 in the plane direction, for example, the outer dimension (outer edge) of the second water absorbing material 47 in the plane direction is used. The outer dimension (outer edge) is measured at, for example, the halfway location of the second water absorbing material 47 in the thickness direction T.

In this way, both end portions of the second base material 46 in the widthwise direction W cover the side surface of the second water absorbing material 47 in the widthwise direction W, and further cover the side surface of the first base material 44 in the widthwise direction W. Thus, the water absorbing material 47 is sealed in the second absorbing layer 42 in the widthwise direction W, and the first water absorbing material 45 is sealed in the first absorbing layer 41 in the widthwise direction W. In another embodiment, both end portions of the first base material 44 in the widthwise direction W cover both end portions of the second base material 46 in the widthwise direction W. That is, the first base material 44 has a pair of overlapping portions P and P that extend along the lengthwise direction L at both end portions in the widthwise direction W. In still another embodiment, the first base material 44, the intermediate layer 43, and the second base material 46 are laminated and joined together in the thickness direction T at both end portions in the widthwise direction W.

In the present embodiment, the first base material 44, the intermediate layer 43, and the second base material 46 are laminated and joined together in the thickness direction T at both end portions of the absorber 14 in the lengthwise direction L. Therefore, the first water absorbing material 45 is sealed in the first absorbing layer 41 by the first base material 44 and the intermediate layer 43 in the lengthwise direction L, and the second water absorbing material 47 is sealed in the second absorbing layer 42 by the second base material 46 and the intermediate layer 43 in the lengthwise direction L. In another embodiment, two end portions of the absorber 14 in the lengthwise direction L are not covered with the first base material 44 and the second base material 46.

In the present embodiment, the intermediate layer 43 is a liquid-permeable sheet and has a substantially rectangular planar shape. The intermediate layer 43 includes an upper layer 43a and a pair of lower layers 43b and 43b that are adjacent to both sides in the widthwise direction W on the surface on non-skin side of the upper layer 43a. The intermediate layer 43 is formed by, in a sheet member having a substantially rectangular planar shape, folding back a pair of side portions that are located on both sides of the central portion in the widthwise direction W toward the non-skin side of the central portion and overlapping the both sides. At that time, the central portion of the sheet member becomes the upper layer 43a, and the pair of side portions of the sheet member become the pair of lower layers 43b and 43b. Therefore, both side portions of the intermediate layer 43 in the widthwise direction W have a two-layer structure in which the upper layer 43a and the lower layer 43b are laminated together and have a substantially rectangular shape extending in the lengthwise direction L. A portion 43d in the vicinity of the center of the intermediate layer 43 in the widthwise direction W has a single-layer structure of the upper layer 43a alone and has a substantially rectangular shape extending along the lengthwise direction L. The upper layer 43a and the lower layers 43b in the two-layer structure are joined together with a pair of heat sealing portions 43c and 43c extending along the lengthwise direction L on both sides of the portion 43d in the widthwise direction W. The heat sealing portion 43c is formed by compressing under heating. In another embodiment, the upper layer 43a and the lower layers 43b in the two-layer structure are joined together with embossed portions extending along the lengthwise direction L on both sides of the portion 43d in the widthwise direction W. The embossed portion is formed by compressing. In still another embodiment, the heat sealing portion and the embossed portion have a shape of a plurality of points or a plurality of curved lines or patterns. In the intermediate layer 43, both end edges in the widthwise direction W are sandwiched between both end portions of the first base material 44 and the second base material 46 in the widthwise direction W, both end edges in the lengthwise direction L are sandwiched between both end portions of the first base material 44 and the second base material 46 in the lengthwise direction L, and the end edges are joined to the end portions, respectively. In another embodiment, both end edges in the lengthwise direction L are not sandwiched between and joined to both end portions of the first base material 44 and the second base material 46 in the lengthwise direction L. Such a two-layer structure makes it possible to temporarily store liquid and to easily secure the time necessary for the superabsorbent polymers to absorb the liquid.

In the present embodiment, the absorber 14 has a pair of channels 48 and 48 extending along the lengthwise direction L. The pair of channels 48 and 48 are band-shaped regions that are located in the second absorbing layer 42, are provided to extend in the lengthwise direction L, and are arranged at a predetermined interval on both sides in the widthwise direction W across the longitudinal centerline CL. The channel 48 is formed so as to straddle the widthwise direction centerline CW in at least the central portion in the lengthwise direction L. The channel 48 that extends along the lengthwise direction L can further increase the rigidity of the absorber 14 in the lengthwise direction L. In another embodiment, the channel 48 is formed so as to reach at least one of both end edges in the lengthwise direction L. The pattern of the pair of channels 48 and 48 in a planar view is, for example, a pattern in which the interval between two channels is constant along the lengthwise direction L. In another embodiment, the pattern of the pair of channels 48 and 48 is, for example, a pattern formed so as to be narrow in the vicinity of the center in the lengthwise direction L and become wider toward both outer sides in the lengthwise direction L. Here, the channel 48 is a region in which the basis weight of the water absorbing material is small compared with a surrounding region of the channel in the absorber 14, for example, a region in which the basis weight of the water absorbing material is small compared with a surrounding region of the channel 48 in the second absorbing layer 42 and includes a case where the basis weight is zero. The channel 48 is formed such that the second base material 46 is recessed toward the intermediate layer 43, that is, the second water absorbing material 47 is recessed toward the intermediate layer 43. In another embodiment, the second water absorbing material 47 is formed so as to be recessed toward the second base material 46. In addition, the pair of channels 48 and 48 overlap the pair of heat sealing portions 43c and 43c in the thickness direction T. In another embodiment, at least a part of the channel 48 does not overlap the heat sealing portion 43c in the thickness direction T. It should be noted that, in another embodiment, the channel 48 extends along the widthwise direction W instead of along the lengthwise direction L or as well as along the lengthwise direction L. In still another embodiment, the number of the channels 48 is one or multiple (three or more). In still another embodiment, the channel 48 is formed in the first absorbing layer 41 instead of in the second absorbing layer 42 or as well as in the second absorbing layer 42. In still another embodiment, the channel 48 is not formed.

As described above, the first water absorbing material 45 and the second water absorbing material 47 contain superabsorbent polymers (SAP). The superabsorbent polymers are not particularly limited as long as the polymers are capable of absorbing and retaining water, and examples thereof include particulate or fibrous superabsorbent polymers. The basis weights of superabsorbent polymers of the first water absorbing material 45 and the second water absorbing material 47 can be appropriately adjusted depending on the absorption performance required for the pants-type diaper 1, and each is, for example, 10 to 500 g/m², and preferably 100 to 400 g/m². One of the first water absorbing material 45 and the second water absorbing material 47 may have a larger basis weight than the other or may have the same basis weight as the other. Here, "the same basis weight" means that the basis weight of one water absorbing material is within a range of ± 30% of the basis weight of the other. The first water absorbing material 45 and the second water absorbing material 47 may further have hydrophilic fibers such as pulp fibers or water-absorbent fibers. The proportion of the superabsorbent polymers in the first water absorbing material 45 and the second water absorbing material 47 is, for example, 80 to 100 mass%, preferably 90 to 100 mass%, and more preferably 95 to 100 mass%. Therefore, the first water absorbing material 45 and the second water absorbing material 47 can be said to contain the superabsorbent polymers as a main component, and the absorber 14 can be said as a so-called SAP sheet. However, the proportion of the superabsorbent polymers in the absorber 14 (including not only the first water absorbing material 45 and the second water absorbing material 47 but also the adhesive, the first base material 44, the intermediate layer 43, and the second base material 46) is, for example, 40 to 80 mass%, preferably 50 to 80 mass%, and more preferably 60 to 80 mass%. In the present embodiment, the first water absorbing material 45 and the second water absorbing material 47 are formed of superabsorbent polymers alone and do not contain any hydrophilic fibers. In another embodiment, the first water absorbing material 45 and the second water absorbing material 47 contain pulp fibers and/or water-absorbent fibers in addition to the superabsorbent polymers.

Examples of the superabsorbent polymers include starch-based, cellulose-based, and synthetic polymer-based macromolecular absorbing agents. Examples of the starch-based or cellulose-based superabsorbent polymers include starch-acrylic acid (salt) graft copolymers, saponified substance of starch-acrylonitrile copolymers, and crosslinked substance of sodium carboxymethyl cellulose. Examples of the synthetic polymer-based superabsorbent polymers include polyacrylate-based, polysulfonate-based, maleate anhydride-based, polyacrylamide-based, polyvinyl alcohol-based, polyethylene oxide-based, polyaspartate-based, polyglutamate-based, polyalginate-based, starch-based, cellulose-based, and other superabsorbent polymers. In the present embodiment, polyacrylate-based (particularly, sodium polyacrylate-based) superabsorbent polymers are preferable. In addition, in the present embodiment, 90 to 100 mass% of the superabsorbent polymers are preferably formed of superabsorbent polymer particles having particle sizes of 150 to 500 µm in the absorber 14. The superabsorbent polymer particles having such a particle size distribution have small and uniform particle sizes and are thus easily retained in the adhesive. The particle sizes of the superabsorbent polymer particles are measured according to the sieving test method described in JIS R 6002: 1998.

The adhesive is not particularly limited as long as the adhesive is capable of fixing the superabsorbent polymers, and examples thereof include a hot melt adhesive. The application pattern of the adhesive is not particularly limited, and examples thereof include a continuous or intermittent omega pattern, a spiral pattern, and a line pattern. The basis weight of the adhesive can be appropriately adjusted such that the liquid absorbing property of the absorber 14 does not significantly degrade and is, for example, 3 to 50 g/m² in each layer. Here, each layer is an adhesive layer between the first water absorbing material 45 and the first base material 44, an adhesive layer between the first water absorbing material 45 and the intermediate layer 43, an adhesive layer between the second water absorbing material 47 and the second base material 46, or an adhesive layer between the second water absorbing material 47 and the intermediate layer 43.

The first base material 44 is not particularly limited as long as the base material is a liquid-permeable sheet. Examples of the first base material 44 include a liquid-permeable non-woven fabric, a hydrophilic non-woven fabric, and a laminated non-woven fabric thereof, and, among them, a highly water-permeable non-woven fabric is preferable. Examples thereof include a spunbonded non-woven fabric and an air-through non-woven fabric formed by polyolefin fibers such as polyethylene (PE) and polypropylene (PP), polyester fibers such as polyethylene terephthalate (PET), polytrimethylene terephthalate (PTT), and polyethylene naphthalate (PEN), and the combination of the above-described fibers. These fibers are preferably fibers on which a hydrophilization treatment has been performed by a well-known method. Alternatively, examples thereof include an airlaid non-woven fabric in which hydrophilic fibers such as pulp fibers or rayon fibers are coated with a hydrophilic binder and a spunlace non-woven fabric in which the above-described hydrophilic fibers and the above-described synthetic fibers are combined together. In the present embodiment, an airlaid non-woven fabric in which liquid-permeable and liquid-retaining pulp fibers are coated with a hydrophilic binder is used. It should be noted that, as the first base material 44, a plurality of layers of one kind or a plurality of kinds of non-woven fabrics out of the above-described non-woven fabrics may be laminated together. The basis weight of the first base material 44 is, for example, 10 to 100 g/m² and preferably 20 to 80 g/m². The thickness of the first base material 44 is, for example, 0.1 to 5 mm and preferably 0.15 to 3 mm.

The second base material 46 is not particularly limited as long as the base material is a water-retaining and liquid-diffusing sheet. Examples of the second base material 46 includes a non-woven fabric formed by synthetic fibers such as polyamide fibers, regenerated fibers such as rayon fibers or acetate fibers, natural fibers such as cotton, silk, hemp, or pulp (cellulose) fibers, and the combination of the above-described fibers. Specific examples thereof include a spunbonded non-woven fabric containing nylon and a spunlace non-woven fabric containing rayon fibers and/or pulp fibers. The spunlace non-woven fabric containing a rayon fiber and/or a pulp fiber may contain a polyolefin fiber and/or a polyester fiber. In the present embodiment, a liquid-retaining and liquid-diffusing spunlace non-woven fabric containing rayon fibers and pulp fibers is used. It should be noted that, as the second base material 46, a plurality of layers of one kind or a plurality of kinds of non-woven fabrics out of the above-described non-woven fabrics may be laminated together. The basis weight of the second base material 46 is, for example, 10 to 200 g/m² and preferably 35 to 150 g/m². The thickness of the second base material 46 is, for example, 0.1 to 5 mm and preferably 0.15 to 3 mm.

The intermediate layer 43 is not particularly limited as long as the layer is a liquid-permeable sheet. As the intermediate layer 43, for example, the same sheet as the first base material 44 can be used. The basis weight of the intermediate layer 43 is, for example, 10 to 100 g/m² and preferably 15 to 80 g/m². The thickness of the intermediate layer 43 is, for example, 0.1 to 5 mm and preferably 0.15 to 3 mm.

In the present embodiment, as illustrated in FIG. 3, the absorber 14 includes a back region BA, a stomach region FA, and a crotch region MA arranged along the lengthwise direction L. Here, the back region BA is a region that overlaps the back portion 3 in the absorber 14 in the thickness direction T. In addition, the stomach region FA is a region that overlaps the stomach portion 2 in the absorber 14 in the thickness direction T. The crotch region MA is a region that is located between the back region BA and the stomach region FA in the absorber 14, that is, a region that overlaps the crotch portion 4 in the thickness direction T. Therefore, the absorber 14 is divided into the back region BA that overlaps the back portion 3, the stomach region FA that overlaps the stomach portion 2, and the crotch region MA that overlaps the crotch portion 4 along the lengthwise direction L.

In the present embodiment, the absorber 14 also includes a high basis weight area 14a, a low basis weight area 14b, and a non-arrangement area 14c. The high basis weight region 14a is a region in which the basis weight of the superabsorbent polymers is high and a region in which the first absorbing layer 41 and the second absorbing layer 42 overlap each other in the thickness direction T. The boundary between the high basis weight region 14a and the low basis weight region 14b is, for example, the outer edge of the first absorbing layer 41. The low basis weight region 14b is a region in which the basis weight of the superabsorbent polymers is low and a region in which the first absorbing layer 41 is not present and only the second absorbing layer 42 is present in the thickness direction T. The boundary between the low basis weight region 14b and the non-arrangement region 14c is, for example, the outer edge of the second absorbing layer 42. The non-arrangement region 14c is a region that does not contain the superabsorbent polymers and a region in which neither the first absorbing layer 41 nor the second absorbing layer 42 is present in the thickness direction T. However, the expression "region that does not contain the superabsorbent polymers" also indicates a case where the superabsorbent polymers are present, but the basis weight thereof is extremely small. The expression "extremely small" means a case where the basis weight of the superabsorbent polymers is 5% or less of the basis weight of the superabsorbent polymers in the high basis weight region 14a. The high basis weight region 14a, the low basis weight region 14b, and the non-arrangement region 14c each has a substantially rectangular shape. However, the shape is not particularly limited, and examples thereof include a rectangular shape having a short side that protrudes in an arc shape, a rectangular shape with rounded corners, an ellipse, and an hourglass. In addition, in a planar view, in the lengthwise direction L and in the widthwise direction W, the low basis weight region 14b is a region that surrounds the periphery of the high basis weight region 14a (at least surrounds three sides) substantially in a frame shape, and the outer dimensions of the low basis weight region 14b are, for example, 5% to 25% larger than the outer dimensions of the high basis weight region 14a. The non-arrangement region 14c is a region that surrounds the periphery of the low basis weight region 14b substantially in a frame shape, and the outer dimensions of the non-arrangement region 14c are, for example, 3% to 15% larger than the outer dimensions of the low basis weight region 14b. In addition, the basis weight of the low basis weight region 14b is, for example, 40% to 60% of the basis weight of the high basis weight region 14a, and the basis weight of the non-arrangement region 14c is, for example, 0% to 5% of the basis weight of the high basis weight region 14a. It should be noted that, in a case where it is difficult to divide the absorber 14 into two regions such as a case where the absorber 14 is one layer not having the first absorbing layer 41 and the second absorbing layer 42, the high basis weight region is defined as a region in which the thickness of the water absorbing material (including the adhesive) is in a range from the maximum value to 50% of the maximum value. The low basis weight portion is the region where the thickness of the water absorbing material (including the adhesive) is in the range of 50% to 5% of the maximum value, and the non-arranged region is the thickness of the water absorbing material (including the adhesive). However, the region is in the range of 5% to 0% of the maximum value. It should be noted that the thickness is the arithmetic mean height (Ra: JIS B 0601-2001). In another embodiment, the absorber 14 does not include at least one of the high basis weight region 14a, the low basis weight region 14b, and the non-arrangement region 14c.

Therefore, in the absorber 14, the back region BA includes a back high basis weight region BAa as the high basis weight region 14a, a back low basis weight region BAb as the low basis weight region 14b, and a back non-arrangement region BAc as the non-arrangement region 14c in the lengthwise direction. In addition, in the absorber 14, the stomach region FA includes a stomach high basis weight region FAa as the high basis weight region 14a and a stomach non-arrangement region FAc as the non-arrangement region 14c in the lengthwise direction. It should be noted that the crotch region MA has only the high basis weight region 14a in the lengthwise direction. Therefore, the basis weights of the superabsorbent polymers in the back high basis weight region BAa and in the stomach high basis weight region FAa are equal to the basis weight of the superabsorbent polymers in the crotch region MA. It should be noted that the expression "equal" means that one basis weight is within a range of 0.7 to 1.3 times (±30%) of the other basis weight. In another embodiment, the stomach region FA includes a stomach low basis weight region FAb as the low basis weight region 14b in addition to the stomach high basis weight region FAa and the stomach non-arrangement region FAc.

Here, the second absorbing layer 42 includes a protrusion portion that protrudes outward more than the end edge of the first absorbing layer 41 at the back end portion in the lengthwise direction L in a planar view. The protrusion portion includes the back low basis weight region BAb, and in the present embodiment, the protrusion portion is the same as the back low basis weight region BAb. The second absorbing layer 42 is covered with the intermediate layer 43, and thus the skin side of the protrusion portion, that is, the back low basis weight region BAb is covered with the intermediate layer 43 and the first base material 44. Furthermore, in the present embodiment, the second absorbing layer 42 includes widthwise protrusion portions that protrude outward more than the end edges of the first absorbing layer 41 at both end portions in the widthwise direction W in a planar view. The skin sides of the widthwise protrusion portions are covered with the intermediate layer 43 and the first base material 44. In another embodiment, the second absorbing layer 42 includes a different protrusion portion that protrudes outward more than the end edge of the first absorbing layer 41 at the front end portion in the lengthwise direction L in a planar view. The different protrusion portion includes the stomach low basis weight region. In still another embodiment, the skin side of the protrusion portion, that is, the back low basis weight region BAb is covered with any one of the intermediate layer 43 and the first base material 44, but is not covered with the other. In still another embodiment, the second absorbing layer 42 does not include the widthwise protrusion portions that protrude outward more than the end edges of the first absorbing layer 41 at both end portions in the widthwise direction W in a planar view.

In the pants-type diaper 1 of the present embodiment, in the absorber 14, the basis weight of the superabsorbent polymers in the crotch region MA is relatively high, and the basis weight of the superabsorbent polymers in the low basis weight region (example: the back low basis weight region BAb) of at least one of the back region BA and the stomach region FA is relatively low. Therefore, a relatively large amount of the superabsorbent polymers are arranged in the crotch region MA in which the absorption of liquid is required, and thus the absorber 14 is capable of securing absorption performance to reliably absorb liquid (example: urine) while preventing leakage. On the other hand, a small amount of the superabsorbent polymers are arranged in the low basis weight region in at least one of the back region BA and the stomach region FA in which the absorption of liquid is not significantly required, whereby the region becomes thin, which makes it possible to suppress the absorber pressing the wearer's skin surface when the stomach portion 2 or the back portion 3 that corresponds to the region is brought into close contact with the skin surface. Thus, the wearer is less likely to feel uncomfortable, and the fitting properties of the pants-type diaper 1 can be improved. Therefore, in the pants-type diaper 1 containing the superabsorbent polymers, the fitting properties can be improved while securing the absorption performance.

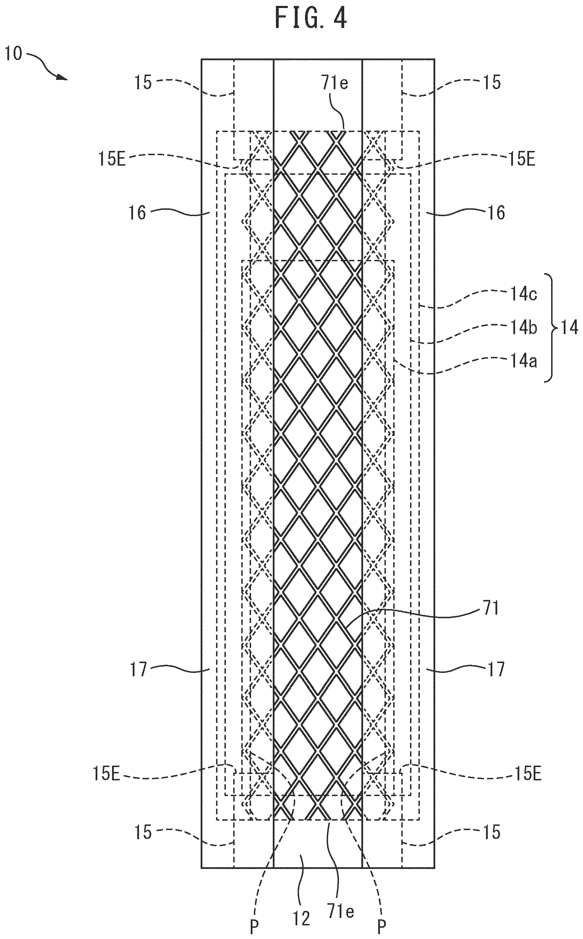

FIG. 4 is a plan view showing a configuration example of the absorbent body 10 according to the embodiment. As shown in the drawing, the absorber 14 of the absorbent body 10 has a compressed portion 71 extending in the lengthwise direction L. The planar shape of the compressed portion 71 is not particularly limited as long as the compressed portion extends in the lengthwise direction L. However, the compressed portion 71 that "extends in the lengthwise direction" includes not only a case where the compressed portion continuously extends in the lengthwise direction L, but also a case where the compressed portion intermittently extend in the lengthwise direction L, another compressed portion overlaps with the intermittent compressed portion in the widthwise direction W, and thus they continuously extend in the lengthwise direction L when seen from the widthwise direction W. The compressed portion 71 exists in the lengthwise direction L, for example, in a range of at least 70% of the length of the absorber 14. The compressed portion 71 exists preferably in a range of 80% or more, and more preferably in a range of 90% or more of the length. In the present embodiment, the compressed portion 71 exists in a range of, for example, 100% of the length of the absorber 14 in the lengthwise direction L. Further, the compressed portion 71 exists in the widthwise direction W, for example, in a range of at least 20% of the width of the absorber 14. The compressed portion preferably exists in a range of 40% or more, and more preferably in a range of 60% or more. In the present embodiment, for example, the compressed portion 71 exists in a range of approximately 70% of the length of the absorber 14 at the substantially central region of the absorber 14 in the widthwise direction W (does not exist in approximately 15% of each of both end portions). However, the range in which the compressed portion 71 exists in the widthwise direction W (hereinafter, also referred to as "compressed region") refers to a region that is formed by sandwiching the compressed portion 71 extending in the lengthwise direction and continuously provided in the widthwise direction W between two virtual straight lines parallel to each other in the lengthwise direction L from both sides in the widthwise direction W, includes the compressed portion 71, and extends in the lengthwise direction L. The compressed portion 71 is a compressed groove formed by compressing a predetermined portion of the absorber 14 from both sides in the thickness direction T with or without heating (for example, embossing). As described above, the compressed portion 71 that extends in the lengthwise direction L is formed in the absorber 14, and thus, the rigidity of the absorber 14 can be increased by the rigidity of the compressed portion 71. It should be noted that in FIGS. 1 to 3 and 5 to 6, the compressed portion 71 is not shown.

In the present embodiment, in the lengthwise direction L, an end edge 71e on the front side (the end portion 2e side of the stomach portion 2) of the compressed portion 71 is positioned on the front side with respect to the end edge on the front side of the leak-proof wall 16. Further, an end edge 71e on the back side (the end portion 2e side of the back portion 3) of the compressed portion 71 is positioned on the back side with respect to the end edge on the back side of the leak-proof wall 16. In other words, in the lengthwise direction L, the end edge 71e on the front side of the compressed portion 71 is positioned on the front side with respect to a back side end edge 15E of the front side fixed region 15. Further, the end edge 71e on the back side of the compressed portion 71 is positioned on the back side with respect to the front side end edge 15E of the back side fixed region 15. Therefore, since the compressed portion 71 is formed so as to extend longer in the lengthwise direction than the leak-proof wall 16, due to the rigidity of the compressed portion 71, the absorber 14 can resist the contraction force of the (elastic member 61 of) the leak-proof wall 16 in the lengthwise direction L.

FIG. 7 is a plan view showing a configuration example of an absorbent body according to the present embodiment. As shown in the drawing, the compressed portion 71 has an inclined grid shape formed of a plurality of rhombic shapes that are elongated in the lengthwise direction L. Therefore, even when the contraction force of the leak-proof wall 16 in the lengthwise direction L is applied to the absorber 14, the direction of the compressed portion 71 is inclined with respect to the direction of the contraction force, that is, the substantially lengthwise direction L, and thus, the contraction force can be laterally released and substantially reduced. Further, although the direction of the compressed portion 71 is inclined with respect to the direction of the compressive force, since the direction is not a direction orthogonal to the direction of the contraction force, that is, the substantially widthwise direction W, the absorber 14 can be prevented from being folded with the compressed portion 71 as a fold line. Therefore, the absorber 14 can be further prevented from being contracted in the lengthwise direction L.

In the present embodiment, in the widthwise direction W, a part of the compressed portion 71 is positioned in a region between the fixed regions 15 on the front side and the back side in the lengthwise direction L. That is, a part of the compressed portion 71 is positioned in a region that overlaps the leak-proof wall 16 in the thickness direction T. Therefore, the rigidity of the region that overlaps the leak-proof wall 16 in the thickness direction T in the absorber 14 can be increased.

In the present embodiment, in the widthwise direction W, a part of the compressed portion 71 is positioned on the outer side with respect to the non-fixed end edge on the inner side (innermost end edge) of the leak-proof wall 16. Therefore, even in this case, the rigidity of the region that overlaps the leak-proof wall 16 in the thickness direction T in the absorber 14 can be increased.

In the present embodiment, both end portions of the second base material 46 in the widthwise direction W, that is, the pair of overlapping portions P and P are joined to overlap both end portions of the first base material 44 in the widthwise direction W. At that time, the joining portion where the overlapping portion P of the second base material 46 and the end portion of the first base material 44 in the widthwise direction W are joined is positioned on the skin side surface of the second water absorbing material 47 that is the absorbent core. The joining portion including the overlapping portion P overlaps a part of the compressed portion 71 in the thickness direction T. In another embodiment, in the absorber 14, conversely, the joining portion where the first absorbent layer 41 is positioned on the non-skin side and the second absorbent layer 42 is positioned on the skin side, and the overlapping portion P of the second base material 46 and the end portion of the first base material 44 in the widthwise direction W are joined is positioned on the non-skin side surface of the second water absorbing material 47 that is the absorbent core. Therefore, since the compressed portion 71 is further formed in the joining portion where the two base materials are laminated to increase rigidity, the rigidity of the region where the joining portion is positioned in the absorber 14 can be increased.

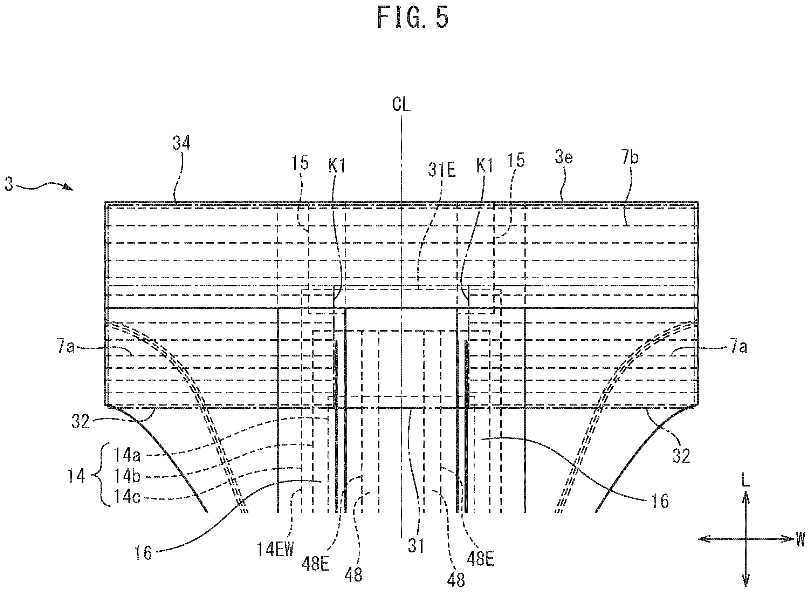

FIG. 5 is an enlarged plan view of a part including the back portion 3 of the pants-type diaper according to the embodiment. In the present embodiment, the back portion 3 has a low stretchable back area 31 having low stretchability in the widthwise direction W and a pair of highly stretchable back areas 32 and 32 having high stretchability in the widthwise direction W. The low stretchable back area 31 is located in the central portion in the widthwise direction W in the back portion 3 and is provided to extend from the front end edge in the lengthwise direction L toward the back side. The pair of highly stretchable back areas 32 and 32 are adjacent to both sides of the low stretchable back area 31 in the widthwise direction W. The low stretchable back area 31 is a region in which the stretch ratio ((length after stretch - original length)/(original length)) with respect to a predetermined tensile force in the widthwise direction W is 1/3 or less of the stretch ratio in the widthwise direction W of the highly stretchable back area 32, preferably a region in which the stretch ratio is 1/5 or less, and more preferably a region in which the stretch ratio is 1/10 or less. For example, the low stretchable back area 31 is a region in which no elastic member that stretches and contracts in the widthwise direction W is present or a region in which an elastic member is present but the elastic member rarely functions as an elastic member (rarely stretches and contracts). On the other hand, the highly stretchable back area 32 is a region in which the elastic members 7a that stretch and contract in the widthwise direction W are present. Therefore, the highly stretchable back area 32 and the low stretchable back area 31 are demarcated by the region in which the elastic members 7a capable of functioning as an elastic member are present and the region in which the elastic members capable of functioning as an elastic member are not present. It should be noted that, the expression "low stretchability" includes a case where the area does not stretch and contract. It should be noted that the stretchability of each stretchable area is determined by the stretchability of the elastic members. The back portion 3 further has a different highly stretchable back area 34 that exhibits high stretchability in the widthwise direction W. The different highly stretchable back area 34 is adjacent to the backs of the pair of highly stretchable back areas 32 and 32 and the low stretchable back area 31 in the lengthwise direction L in a planar view. The different highly stretchable back area 34 is a region in which the stretch ratio in the widthwise direction W is substantially the same as the stretch ratio in the widthwise direction W of the highly stretchable back area 32. The different highly stretchable back area 34 is demarcated as a region in which the elastic members 7b are present. Therefore, the end portion on the back side of the back portion 3 in the lengthwise direction L is easily pressed against the wearer's back by the different highly stretchable back area 34. The elastic members 7b are positioned on the back side (back portion 3 side) in the lengthwise direction L with respect to the absorber 14.

In the present embodiment, the portions on the back side of the channels 48 in the lengthwise direction L overlap the low stretchable back area 31 in the thickness direction T. In addition, in a planar view, an inner end edge K1 in the widthwise direction W of each of the pair of highly stretchable back areas 32 and 32 is located between an end edge 14EW of the absorber 14 that faces the highly stretchable back area 32 in the widthwise direction W and (an end edge 48E in the widthwise direction W of) the channel 48. The end edge K1 is generally the inner end edge of the elastic members 7a in the widthwise direction W. Therefore, in the absorber 14 including the back portion of the channel 48 in the lengthwise direction L, the central portion in the widthwise direction W is rarely stretched in the widthwise direction W due to the low stretchable back area 31, and both end portions in the widthwise direction W are stretched in the widthwise direction W due to the pair of highly stretchable back areas 32 and 32.

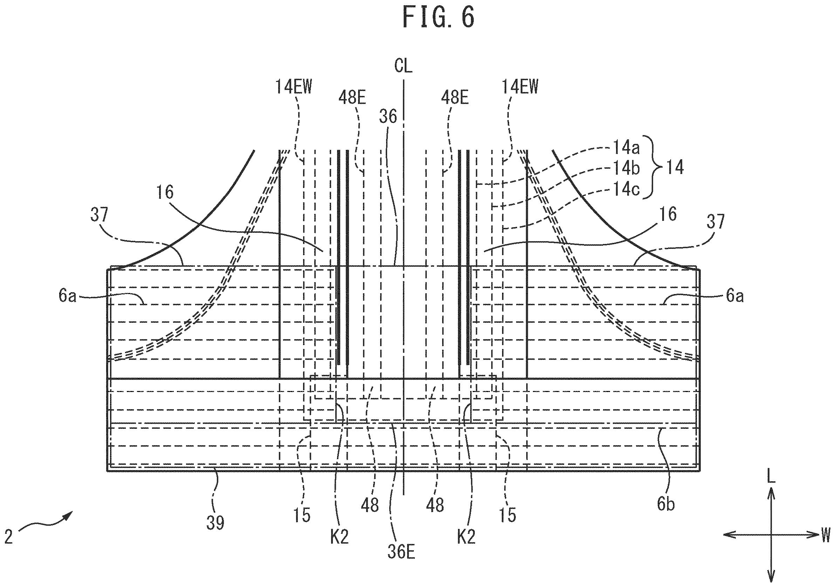

FIG. 6 is an enlarged plan view of a part including the stomach portion 2 of the pants-type diaper according to the embodiment. In the present embodiment, the stomach portion 2 is substantially the same as the back portion 3. That is, the stomach portion 2 has a low stretchable stomach area 36 having low stretchability in the widthwise direction W and a pair of highly stretchable stomach areas 37 and 37 having high stretchability in the widthwise direction W. The low stretchable stomach area 36 is located in the central portion in the widthwise direction W in the stomach portion 2 and is provided to extend from the back end edge in the lengthwise direction L toward the front side. The pair of highly stretchable stomach areas 37 and 37 are adjacent to both sides of the low stretchable stomach area 36 in the widthwise direction W. The low stretchable stomach area 36 is a region in which the stretch ratio ((length after stretch - original length)/(original length)) with respect to a predetermined tensile force in the widthwise direction W is 1/3 or less of the stretch ratio in the widthwise direction W of the highly stretchable stomach area 37, preferably a region in which the stretch ratio is 1/5 or less, and more preferably a region in which the stretch ratio is 1/10 or less. For example, the low stretchable stomach area 36 is a region in which no elastic member that stretches and contracts in the widthwise direction W is present or a region in which an elastic member is present but the elastic member rarely functions as an elastic member (rarely stretches and contracts). On the other hand, the highly stretchable stomach area 37 is a region in which the elastic members 6a that stretch and contract in the widthwise direction W are present. Therefore, the highly stretchable stomach area 37 and the low stretchable stomach area 36 are demarcated by the region in which the elastic members 6a capable of functioning as an elastic member are present and the region in which the elastic members capable of functioning as an elastic member are not present. It should be noted that the stretchability of each stretchable area is determined by the stretchability of the elastic members. The stomach portion 2 further has a different highly stretchable stomach area 39 that exhibits high stretchability in the widthwise direction W. The different highly stretchable stomach area 39 is adjacent to the front sides of the pair of highly stretchable stomach areas 37 and 37 and the low stretchable stomach area 36 in the lengthwise direction L. The different highly stretchable stomach area 39 is a region in which the stretch ratio in the widthwise direction W is substantially the same as the stretch ratio in the widthwise direction W of the highly stretchable stomach area 37. The different highly stretchable stomach area 39 is demarcated as a region in which the elastic members 6b are present. Therefore, the front end portion of the stomach portion 2 in the lengthwise direction L is easily pressed against the wearer's abdomen by the different highly stretchable stomach area 39. The elastic members 6b are positioned on the front side (stomach portion 2 side) in the lengthwise direction L with respect to the absorber 14.

In the present embodiment, the front portions of the channels 48 in the lengthwise direction L overlap the low stretchable stomach area 36 in the thickness direction T. In addition, in a planar view, an inner end edge K2 in the widthwise direction W of each of the pair of highly stretchable stomach areas 37 and 37 is located between an end edge 14EW of the absorber 14 that faces the highly stretchable stomach area 37 in the widthwise direction W and (an end edge 48E in the widthwise direction W of) the channel 48. The end edge K2 is generally the inner end edge of the elastic members 6a in the widthwise direction W. Therefore, in the absorber 14 including the front portion of the channel 48 in the lengthwise direction L, the central portion in the widthwise direction W is rarely stretched and contracted in the widthwise direction W due to the low stretchable front area 36, and both end portions in the widthwise direction W are stretched in the widthwise direction W due to the pair of highly stretchable stomach areas 37 and 37.

In the pants-type diaper 1 of the present embodiment, the channel 48 of the absorber 14 reaches at least one of the back portion 3 (back waist belt) and the stomach portion 2 (stomach waist belt) in the lengthwise direction L. Therefore, liquid (example: urine) can be transported and distributed to the end portion on the back side (the back side in the lengthwise direction L) and/or to the end portion on the stomach side (the front side in the lengthwise direction L) of the absorber 14. This makes it possible for the superabsorbent polymers in the end portions on the back side and/or the front side of the absorber 14 in the lengthwise direction L, which is hard to contribute to absorption, to absorb liquid and makes it possible to effectively use the superabsorbent polymers. Then, in this pants-type diaper 1, the inner end portion in the widthwise direction W in at least one of the highly stretchable area (highly stretchable back area 32 and highly stretchable stomach area 37) of the back portion 3 and the stomach portion 2 is located between the edge 14WE facing the highly stretchable area in the widthwise direction W of the absorber 14 and the channel 48. That is, the central portion of the absorber 14 in the widthwise direction W overlaps the low stretchable areas (low stretchable back area 31 and low stretchable stomach area 36) in the thickness direction T. Therefore, a contractive force that contracts the absorber 14 in the widthwise direction W is not too strong, and thus an event that the back end portion of the absorber 14 in the lengthwise direction L contracts in the widthwise direction W and becomes a convex shape before the diaper is worn can be suppressed. In addition, both side portions of the absorber 14 in the widthwise direction W overlap the highly stretchable areas in the thickness direction T. Therefore, the absorber 14 can be pulled in the widthwise direction W from both sides of the channels 48 due to the contractive forces of the highly stretchable areas. This makes it possible to suppress an event that the end portion of the absorber 14 in the lengthwise direction L becomes a convex shape and the end portion of the absorbent body 10 in the lengthwise direction L detaches from the wearer's body. That is, the flatness in at least one of the back portion 3 and the stomach portion 2 can be maintained before and after the diaper is worn. This makes it possible to suppress a situation in which liquid leaks through a space generated between at least one of the back portion 3 and the stomach portion 2 and the skin surface. This makes it possible to suppress the leakage of liquid while suppressing the deterioration in the fitting properties of the pants-type diaper 1.

Next, a producing method of the absorber 14 will be described. First, a hot melt adhesive is used to coat a sheet for the second base material 46 while moving the sheet for the second base material 46 in the lengthwise direction L. Next, superabsorbent polymers are sprayed along the lengthwise direction L from a superabsorbent polymer supply device onto both end portions and the central portion in the widthwise direction W of the sheet for the second base material 46 coated with the hot melt adhesive, respectively. At this time, a small amount of the superabsorbent polymers migrate to regions (corresponding to the channels) between both end portions and the central portion. Next, a sheet for the intermediate layer 43 coated with a hot melt adhesive on both surfaces is laminated on the super absorbent polymers on the sheet for the second base material 46. Next, superabsorbent polymers are sprayed along the lengthwise direction L from a different superabsorbent polymer supply device onto the central portion in the widthwise direction W of the sheet for the intermediate layer 43 coated with the hot melt adhesive while moving the laminate in the lengthwise direction L. Next, a sheet for the first base material 44 coated with a hot melt adhesive is laminated on the superabsorbent polymers on the sheet for the intermediate layer 43 with the hot melt adhesive facing the superabsorbent polymers. In addition, both side portions of the sheet for the second base material 46 in the widthwise direction W are folded back toward above both side portions of the sheet for the first base material 44 in the widthwise direction W, thereby obtaining a laminate. Thereafter, the laminate is allowed to pass through a pair of embossing rolls and a pair of press rolls, that is, embossing is performed, and the thickness of the laminate is adjusted by pressing, thereby obtaining the absorber 14. The order of the pair of embossing rolls and the pair of press rolls may be reversed. Alternatively, a pair of rolls in which the pair of embossing rolls and the pair of press rolls are integrated together may be used.

Next, a method for manufacturing a pants-type diaper using the above-described absorbent body 14 will be described. The liquid-permeable top sheet 12 is attached to the upper surface (the surface of the first base material 44) of the absorber 14 produced as described above, and the liquid-impermeable back sheet 13 is attached to the lower surface (the surface of the second base material 46) of the absorber 14, thereby obtaining a laminated body. Next, the leak-proof wall 16-attached side sheets 17 are attached to both sides of the laminated body in the widthwise direction W, thereby obtaining the absorbent body 10. After that, the absorbent body 10 is attached onto the cover sheet 5 (including the elastic members 6, 7, and 8), and the both end portions 2a and 2a of the stomach portion 2 in the widthwise direction W and both end portions 3a and 3a of the back portion 3 in the widthwise direction W are joined together. Thus, the pants-type diaper 1 is manufactured.

Next, the advantageous effects of the pants-type diaper 1 (absorbent article) according to the embodiment will be described. In the pants-type diaper 1, in the lengthwise direction L, the end edge 71e on the front side of the compressed portion 71 (end portion 2e side of the stomach portion 2) is positioned on the front side with respect to the end edge on the front side of the leak-proof wall 16 (end edge 15E of the front side fixed region 15), and the end edge on the back side of the compressed portion (end portion 3e side of the back portion 3) is positioned on the back side with respect to the end edge on the back side of the leak-proof wall 16 (end edge 15E of the back side fixed region 15). As described above, since the compressed portion 71 is formed to extend in the lengthwise direction L longer than the leak-proof wall 16 and to exist up to a position on the outer side of the leak-proof wall 16 in the lengthwise direction L, due to the rigidity of the compressed portion 71, the absorber 14 can resist the contraction force of (the elastic member 61 of) the leak-proof wall 16 in the lengthwise direction L. Thus, the absorber 14 can be prevented from being contracted in the lengthwise direction L, and the entire pants-type diaper 1 can be prevented from being contracted in the lengthwise direction L by being dragged by the contraction of the absorber 14. Therefore, the generation of wrinkles in the pants-type diaper 1 can be suppressed, and the deterioration in the fitting properties and the deterioration in the absorption performance of the pants-type diaper 1 can be suppressed.

In a reference example which does not form part of the present invention, the compressed portion 71 has a wavy shape that alternately protrudes toward one side and the other side of the widthwise direction W while extending in the lengthwise direction L. Here, in order to increase the rigidity of the absorber 14 to oppose the compressive force of the leak-proof wall 16 in the lengthwise direction L, it is considered that the area of the compressed region may be increased. For example, in order to increase the area of the compressed region, the area (in a planar view) of the compressed portion 71 may be increased. However, when the area of the compressed portion 71 is excessively increased, there is a risk that the rigidity of the absorber 14 becomes excessively high, that is, the absorber becomes excessively hard, which may deteriorate the fitting properties or increase the discomfort of the wearer. Therefore, in the pants-type diaper 1 (absorbent article), the compressed portion 71 has a wavy shape that extends in the lengthwise direction L. Thus, the area of the compressed region can be increased while suppressing an increase in the area of the compressed portion 71. In other words, the area of the compressed region can be increased while suppressing the area ratio, which is a ratio of the area of the compressed portion 71 to the area of the compressed region, at a low level. Therefore, the rigidity of the absorber 14 can be increased to oppose the compressive force of the leak-proof wall 16 in the lengthwise direction L. Thus, the absorber 14 can be prevented from being contracted in the lengthwise direction, and the entire pants-type diaper 1 can be prevented from being contracted in the lengthwise direction L by being dragged by the contraction of the absorber 14. Therefore, the generation of wrinkles in the pants-type diaper 1 can be suppressed, and the deterioration in the fitting properties and the deterioration in the absorption performance of the pants-type diaper 1 can be suppressed.

As a preferable aspect of the present embodiment, in the widthwise direction W, a part of the compressed portion 71 is positioned in a region between the fixed regions 15 and 15 on the front side and the back side in the lengthwise direction L. That is, a part of the compressed portion 71 is positioned in the region that overlaps the leak-proof wall 16 in the thickness direction T. Therefore, due to the rigidity of the compressed portion 71, the absorber 14 can more strongly resist the contraction force of the leak-proof wall 16 in the lengthwise direction L. Therefore, the absorber 14 can be further prevented from being contracted in the lengthwise direction L.

As a preferable aspect of the present embodiment, in the widthwise direction W, a part of the compressed portion 71 is positioned on the outer side with respect to the non-fixed end edge on the inner side of the leak-proof wall 16. That is, a part of the compressed portion 71 is positioned in a region that further overlaps the leak-proof wall 16 in the region that overlaps the leak-proof wall 16 in the thickness direction T. Therefore, due to the rigidity of the compressed portion 71, the absorber 14 can more strongly resist the contraction force of the leak-proof walls 16 in the lengthwise direction L. Therefore, the absorber 14 can be further prevented from being contracted in the lengthwise direction L.

As a preferable aspect of the present embodiment, the absorber 14 has a rectangular shape in a planar view. Here, if the absorber 14 has an hourglass shape, that is, a shape having a portion narrowed in the widthwise direction W at the central portion in the lengthwise direction L, the contraction force in the lengthwise direction L due to the leak-proof wall 16 will be buffered, and the absorber 14 will be less likely to be contracted in the lengthwise direction L. However, in the pants-type diaper 1, since the absorber 14 has a rectangular shape in a planar view, the absorber 14 is easily contracted in the lengthwise direction L due to the contraction force of the leak-proof wall 16 in the lengthwise direction L. That is, there is a risk that wrinkles are generated in the pants-type diaper 1 to deteriorate the fitting properties and deteriorate the absorption performance of the pants-type diaper 1. However, in the pants-type diaper 1, since the compressed portion 71 has each of the above-described configurations such as a configuration in which the compressed portion extends longer in the lengthwise direction L than at least the leak-proof wall16, due to the rigidity of the compressed portion, the absorber can resist the contraction force of the leak-proof wall in the lengthwise direction, which makes it possible to prevent the absorber from being contracted in the lengthwise direction.

In a preferable aspect of the present embodiment, the contraction force of the leak-proof wall 16, that is, the contraction force of the elastic member 61, is lower than the contraction force of the leg gather (leg gather), that is, the elastic member 8. Therefore, due to the rigidity of the compressed portion 71, the absorber 14 can further oppose the contraction force of the leak-proof wall 16 in the lengthwise direction L, which makes it possible to further prevent the absorber 14 from being contracted in the lengthwise direction L.

As a preferable aspect of the present embodiment, in the absorber 14, the pair of overlapping portions P and P at both end portions in the widthwise direction W in the second base material 46 (second core wrap) are joined to both end portions of the first base material 44 (first core wrap) in the widthwise direction W at a pair of joining portions in the thickness direction T. Then, the joining portion overlaps a part of the compressed portion 71 in the thickness direction T. Therefore, the rigidity of the compressed portion 71 can be further increased. Therefore, due to the rigidity of the compressed portion 71, the absorber 14 can resist the contraction force of the leak-proof wall 16 force in the lengthwise direction L, which makes it possible to further prevent the absorber 14 from being contracted in the lengthwise direction L.

In a preferable aspect of the present embodiment, the absorber 14 has at least one channel 48 that extends along the lengthwise direction L. Therefore, slight bending in the widthwise direction easily occurs in the absorber 14 from the channel 48 extending along the lengthwise direction L as a bending point, and the rigidity in the lengthwise direction L can be further increased. Therefore, the absorber 14 can further oppose the contraction force of the leak-proof wall 16 in the lengthwise direction L, which makes it possible to further prevent the absorber 14 from being contracted in the lengthwise direction L.

In a preferable aspect of the present embodiment, the top sheet 12 includes a plurality of protruding portions and a plurality of recessed portions that extend in the lengthwise direction L (not shown). Therefore, the absorbent body 10 can increase the rigidity in the lengthwise direction L. Therefore, by adding the rigidity of the top sheet 12, the absorber can further oppose the contraction force of the leak-proof wall 16 in the lengthwise direction L, which makes it possible to further prevent the absorber 14 from being contracted in the lengthwise direction L.

As another embodiment, while not shown, the disposable diaper 1 is a tape-type diaper. The tape-type diaper includes a pair of locking members that are located on both outer sides in the widthwise direction W in the back portion of the absorbent body 10. The tape-type diaper includes a stretching/contracting member preferably at the center in the widthwise direction W in the back portion of the absorbent body 10 so as to straddle the lengthwise direction centerline CL and more preferably between the pair of locking members. The configuration of the absorbent body 10 is as described above. Even in this case, the individual effects described above can be exhibited.

The absorbent article and the absorber of the present invention are not limited to the above-described embodiments, and the embodiments can be combined or can be applied to well-known techniques without departing from the scope of the object and the subject matter of the present invention.

### REFERENCE SIGNS LIST

1 pants-type diaper
2 stomach portion
3 back portion
10 absorbent body
14 absorber
15 fixation region
17 side seat
61 elastic member
16 leak-proof wall
71 compressed portion
71e end edge

## Claims

1. An absorbent article (1) having a lengthwise direction, a widthwise direction, and a thickness direction that are orthogonal to each other, and comprising: a stomach portion (2); a back portion (3); and an absorbent body (10) that is positioned between the stomach portion (2) and the back portion (3) and includes an absorber (14), wherein
the absorber (14) contains superabsorbent polymers and
includes a compressed portion (71) that extends in the lengthwise direction,
the absorbent body (10) includes a pair of side sheets (17) that are positioned on both sides in the widthwise direction and extend in the lengthwise direction on a surface on a skin side,
the pair of side sheets (17) each has
fixed regions (15) that are positioned at end portions of each of the side sheets (17) on a front side and a back side in the lengthwise direction and fixed to a surface of the absorbent body (10), and
a leak-proof wall (16) that includes an elastic member (61) extending along the lengthwise direction, positioned between the fixed regions (15) on the front side and the back side of each of the side sheets (17) in the lengthwise direction, and has an end edge on an outer side in the widthwise direction which is fixed to the surface of the absorbent body (10) and an end edge on an inner side in the widthwise direction which is not fixed, and
in the lengthwise direction, an end edge (71e) on the front side of the compressed portion (71) is positioned on the front side with respect to an end edge on the front side of the leak-proof wall (16), and an end edge (71e) on the back side of the compressed portion (71) is positioned on the back side with respect to an end edge on the back side of the leak-proof wall (16), wherein
the compressed portion (71) has an inclined grid shape formed of a plurality of rhombic shapes that are elongated in the lengthwise direction, the direction of the compressed portion (71) being inclined with respect to the lengthwise direction;
wherein at each of both sides in the widthwise direction, a part of the compressed portion (71) is positioned in a region between the fixed regions (15) on the front side and the back side in the lengthwise direction.

2. The absorbent article (1) according to claim 1, wherein
in the widthwise direction, a part of the compressed portion (71) is positioned on an outer side with respect to the non-fixed end edge on an inner side of the leak-proof wall (16).

3. The absorbent article (1) according to claim 1 or 2, wherein
the absorber (14) has a rectangular shape in a planar view.

4. The absorbent article (1) according to any one of claims 1 to 3, further comprising:
a crotch portion (4) positioned between the stomach portion (2) and the back portion (3), wherein
the crotch portion (4) includes leg gathers in portions that surround both leg portions of a wearer, and
a contraction force of the leak-proof wall (16) is lower than a contraction force of the leg gathers.

5. The absorbent article (1) according to any one of claims 1 to 4, wherein
the absorber (14) includes:
an absorbent core containing superabsorbent polymers,
a first core wrap that covers a surface on a skin side of the absorbent core, and
a second core wrap that covers a surface on a non-skin side of the absorbent core,
each of both end portions of the first core wrap and each of both end portions of the second core wrap in the widthwise direction are joined in a joining portion extending along the lengthwise direction,
the joining portion is positioned on one of the surface on the skin side or the surface on the non-skin side of the absorbent core, and
the joining portion overlaps a part of the compressed portion (71) in the thickness direction.

6. The absorbent article (1) according to any one of claims 1 to 5, wherein
the absorber (14) has at least one channel (48) extending along the lengthwise direction.

7. The absorbent article (1) according to any one of claims 1 to 6, wherein
the absorbent body (10) includes a top sheet (12) positioned on a skin side of the absorber (14), and
the top sheet (12) includes:
a plurality of protruding portions extending along the lengthwise direction and arranged side by side at intervals, and
a plurality of recessed portions each positioned between adjacent protruding portions of the plurality of protruding portions.

## Patentansprüche

1. Absorbierender Artikel (1), der eine Längsrichtung, eine Breitenrichtung und eine Dickenrichtung aufweist, die rechtwinklig zueinander vorliegen, und der Folgendes umfasst: einen Bauchteil (2), einen Rückenteil (3), und einen Saugkörper (10), der zwischen dem Bauchteil (2) und dem Rückenteil (3) positioniert ist und ein Saugelement (14) einschließt, wobei
das Saugelement (14) superabsorbierende Polymere enthält und
einen komprimierten Teil (71) einschließt, der sich in der Längsrichtung erstreckt,
der Saugkörper (10) ein Paar von Seitenlagen (17) einschließt, die auf beiden Seiten in der Breitenrichtung positioniert sind und sich in der Längsrichtung auf einer Oberfläche auf einer Hautseite erstrecken,
das Paar von Seitenlagen (17) jeweils Folgendes aufweist:
fixierte Bereiche (15), die an Endteilen von jeder der Seitenlagen (17) auf einer Vorderseite und einer Rückenseite in der Längsrichtung positioniert sind und zu einer Oberfläche des Saugkörpers (10) fixiert sind, und
eine auslaufsichere Wand (16), die ein elastisches Element (61) einschließt, sich entlang der Längsrichtung erstreckt, zwischen den fixierten Bereichen (15) auf der Vorderseite und der Rückenseite von jeder der Seitenlagen (17) in der Längsrichtung positioniert ist und Folgendes aufweist: einen Endrand auf einer äußeren Seite in der Breitenrichtung, der an der Oberfläche des Saugkörpers (10) fixiert ist, und einen Endrand auf einer inneren Seite in der Breitenrichtung, der nicht fixiert ist, und
in der Längsrichtung ein Endrand (71e) auf der Vorderseite des komprimierten Teils (71) auf der Vorderseite in Bezug auf einen Endrand auf der Vorderseite der auslaufsicheren Wand (16) positioniert ist und ein Endrand (71e) auf der Rückenseite des komprimierten Teils (71) auf der Rückenseite in Bezug auf einen Endrand auf der Rückenseite der auslaufsicheren Wand (16) positioniert ist, wobei
der komprimierte Teil (71) eine schräge Gitterform aufweist, die aus einer Vielzahl von rhombischen Formen ausgebildet ist, die in der Längsrichtung langgestreckt sind, wobei die Richtung des komprimierten Teils (71) in Bezug auf die Längsrichtung schräg ist;
wobei an jeder der beiden Seiten in der Breitenrichtung ein Teil des komprimierten Teils (71) in einem Bereich zwischen den fixierten Bereichen (15) auf der Vorderseite und der Rückenseite in der Längsrichtung positioniert ist.

2. Absorbierender Artikel (1) nach Anspruch 1, wobei
in der Breitenrichtung ein Teil des komprimierten Teils (71) auf einer äußeren Seite in Bezug auf den nicht-fixierten Endrand auf einer inneren Seite der auslaufsicheren Wand (16) positioniert ist.

3. Absorbierender Artikel (1) nach Anspruch 1 oder 2, wobei das Saugelement (14) eine rechteckige Form in einer Draufsicht aufweist.

4. Absorbierender Artikel (1) nach einem der Ansprüche 1 bis 3, der weiter Folgendes umfasst:
einen Schrittteil (4), der zwischen dem Bauchteil (2) und dem Rückenteil (3) positioniert ist, wobei
der Schrittteil (4) Beinraffungen in Teilen einschließt, die beide Beinteile eines Trägers umgeben, und
eine Kontraktionskraft der auslaufsicheren Wand (16) geringer ist als eine Kontraktionskraft der Beinraffungen.

5. Absorbierender Artikel (1) nach einem der Ansprüche 1 bis 4, wobei das Saugelement (14) Folgendes einschließt:
einen Saugkern, der superabsorbierende Polymere enthält,
eine erste Kernumhüllung, die eine Oberfläche auf einer Hautseite des Saugkerns bedeckt, und
eine zweite Kernumhüllung, die eine Oberfläche auf einer Nicht-Hautseite des Saugkerns bedeckt,
jeder der beiden Endteile der ersten Kernumhüllung und jeder der beiden Endteile der zweiten Kernumhüllung in der Breitenrichtung in einem Verbindungteil verbunden sind, der sich entlang der Längsrichtung erstreckt,
der Verbindungteil auf einer der Oberfläche auf der Hautseite oder der Oberfläche auf der Nicht-Hautseite des Saugkerns positioniert ist und
der Verbindungteil mit einem Teil des komprimierten Teils (71) in der Dickenrichtung überlappt.

6. Absorbierender Artikel (1) nach einem der Ansprüche 1 bis 5, wobei
das Saugelement (14) mindestens einen Kanal (48) aufweist, der sich entlang der Längsrichtung erstreckt.

7. Absorbierender Artikel (1) nach einem der Ansprüche 1 bis 6, wobei
der Saugkörper (10) eine obere Lage (12) einschließt, die auf einer Hautseite des Saugelements (14) positioniert ist, und
die obere Lage (12) Folgendes einschließt:
eine Vielzahl von vorstehenden Teilen, die sich entlang der Längsrichtung erstrecken und nebeneinander in Abständen angeordnet sind, und
eine Vielzahl von vertieften Teilen, die jeweils zwischen benachbarten vorstehenden Teilen der Vielzahl von vorstehenden Teilen positioniert sind.

## Revendications

1. Article absorbant (1) présentant une direction de longueur, une direction de largeur et une direction d'épaisseur qui sont orthogonales les unes par rapport aux autres et comprenant : une partie ventrale (2) ; une partie dorsale (3) ; et un corps absorbant (10) qui est positionné entre la partie ventrale (2) et la partie dorsale (3) et inclut un absorbant (14), où
l'absorbant (14) contient des polymères superabsorbants et inclut une partie comprimée (71) qui se prolonge dans la direction de longueur,
le corps absorbant (10) inclut une paire de feuillets latéraux (17) qui sont positionnés des deux côtés dans la direction de largeur et qui se prolongent dans la direction de longueur sur une surface sur un côté peau,
les feuillets latéraux de la paire (17) présentent chacun
des zones fixes (15) qui sont positionnées au niveau des parties d'extrémité de chacun des feuillets latéraux (17) sur un côté avant et un côté arrière dans la direction de longueur et qui sont fixées à une surface du corps absorbant (10) et
une paroi étanche aux fuites (16) incluant un organe élastique (61) qui se prolonge le long de la direction de longueur, qui est positionnée entre les zones fixes (15) du côté avant et du côté arrière de chacun des feuillets latéraux (17) dans la direction de longueur et qui présente une bordure d'extrémité fixée à la surface du corps absorbant (10) sur un côté extérieur dans la direction de largeur et une bordure d'extrémité non fixée sur un côté intérieur dans la direction de largeur et
dans la direction de longueur, une bordure d'extrémité (71e) sur le côté avant de la partie comprimée (71) est positionnée sur le côté avant par rapport à une bordure d'extrémité sur un côté avant de la paroi étanche aux fuites (16), et une bordure d'extrémité (71e) sur le côté arrière de la partie comprimée (71) est positionnée sur le côté arrière par rapport à une bordure d'extrémité sur un côté arrière de la paroi étanche aux fuites (16),
où la partie comprimée (71) a une forme de grille inclinée constituée d'une pluralité de formes rhombiques qui sont allongées dans la direction de longueur, la direction de la partie comprimée (71) étant inclinée par rapport à la direction de longueur ;
où, au niveau de chacun des deux côtés dans la direction de largeur, une section de la partie comprimée (71) est positionnée dans une zone située entre les zones fixes (15) sur le côté avant et le côté arrière dans la direction de longueur.

2. Article absorbant (1) selon la revendication 1, où
dans la direction de largeur, une section de la partie comprimée (71) est positionnée sur un côté extérieur par rapport à la bordure d'extrémité non fixée sur un côté intérieur de la paroi étanche aux fuites (16).

3. Article absorbant (1) selon la revendication 1 ou 2, où
l'absorbant (14) a une forme rectangulaire en vue en plan.

4. Article absorbant (1) selon l'une quelconque des revendications 1 à 3 comprenant en outre :
une partie correspondant à l'entrejambe (4) positionnée entre la partie ventrale (2) et la partie dorsale (3), où
la partie correspondant à l'entrejambe (4) inclut des fronces dans des sections qui entourent les deux parties correspondant aux jambes d'un porteur et
une force de contraction de la paroi étanche aux fuites (16) est plus basse qu'une force de contraction des fronces situées au niveau des jambes.

5. Article absorbant (1) selon l'une quelconque des revendications 1 à 4, où l'absorbant (14) inclut :
un noyau absorbant contenant des polymères superabsorbants,
une première enveloppe du noyau qui couvre une surface sur un côté peau du noyau absorbant et
une seconde enveloppe du noyau qui couvre une surface sur un côté opposé à la peau du noyau absorbant,
chacune des deux parties d'extrémité de la première enveloppe du noyau et chacune des deux parties d'extrémité de la seconde enveloppe du noyau sont jointes dans la direction de largeur au niveau d'une partie de jonction qui se prolonge le long de la direction de longueur,
la partie de jonction est positionnée sur une parmi la surface sur le côté peau et la surface sur le côté opposé à la peau du noyau absorbant et
la partie de jonction chevauche une section de la partie comprimée (71) dans la direction d'épaisseur.

6. Article absorbant (1) selon l'une quelconque des revendications 1 à 5, où
l'absorbant (14) comporte au moins un sillon (48) qui se prolonge le long de la direction de longueur.

7. Article absorbant (1) selon l'une quelconque des revendications 1 à 6, où
le corps absorbant (10) inclut un feuillet supérieur (12) positionné d'un côté peau de l'absorbant (14) et
le feuillet supérieur (12) inclut :
une pluralité de parties en saillie qui se prolongent le long de la direction de longueur et sont agencées côte à côte à certains intervalles et
une pluralité de parties en retrait chacune positionnée entre des parties en saillie adjacentes de la pluralité de parties en saillie.
